# EUROPEAN PATENT SPECIFICATION

(11) **EP 1 420 778 B1**
(45) Date of publication and mention of the grant of the patent: **22.11.2006**
(21) Application number: 02718181.7
(22) Date of filing: 06.03.2002
(51) Int. Cl.: A61K 31/166, A61K 31/145, A61P 31/14, A61P 7/04

(54) **USE OF MEK INHIBITORS FOR TREATING VIRUS INDUCED HEMORRHAGIC SHOCK OR FEVER**
VERWENDUNG VON MEK HEMMERN ZUR BEHANDLUNG VON VIRUSVERMITTELTEM HÄMORRAGISCHEM SCHOCK ODER FIEBER
UTILISATION D'INHIBITEURS DE LA KINASE POUR TRAITER SHOCK HEMORRHAGIQUE VIRAL OU FIÈVRE

(30) Priority: 06.03.2001 EP 01105552; 12.03.2001 US 274647 P
(43) Date of publication of application: 26.05.2004
(73) Proprietor: Bevec, Dorian, 82110 Germering (DE)
(72) Inventor: BEVEC, Dorian, 81369 Munich (DE); STROHER, Ute, Winnipeg, Manitoba R3N 1C1 (CA); KLENK, Hans-Dieter, 35440 Linden (DE); WALLASCH, Christian, 81375 Munich (DE)
(74) Representative: Salgo, Reinhold Caspar
(86) International application number: PCT/EP2002/002467
(87) International publication number: WO 2002/069960

(56) References cited:
- WO-A-00/40237
- WO-A-00/56706
- WO-A-00/56725
- WO-A-98/37881
- US-A- 5 525 625
- US-A- 6 147 107
- JAFFEE BRUCE D ET AL: "Inhibition of MAP kinase kinase (MEK) results in an anti-inflammatory response in vivo." BIOCHEMICAL AND BIOPHYSICAL RESEARCH COMMUNICATIONS., vol. 268, no. 2, 16 February 2000 (2000-02-16), pages 647-651, XP001088905 ISSN: 0006-291X
- WARRIOR USHA ET AL: "Development of a p38 kinase binding assay for high throughput screening." JOURNAL OF BIOMOLECULAR SCREENING, vol. 4, no. 3, June 1999 (1999-06), pages 129-135, XP001088800 ISSN: 1087-0571
- SMITH C C ET AL: "RAS-BINDING AND PHOSPHORYLATION BY HERPES SIMPLEX VIRUS TYPE 2 RR1 PK (ICP10) AND ACTIVATION OF THE RAS/MEK/MAPK MITOGENIC PATHWAY ARE REQUIRED FOR TIMELY ONSET OF VIRUS GROWTH" JOURNAL OF VIROLOGY, THE AMERICAN SOCIETY FOR MICROBIOLOGY, US, vol. 74, no. 22, November 2000 (2000-11), pages 10417-10429, XP002185543 ISSN: 0022-538X
- SCHNITTLER H-J ET AL: "REPLICATION OF MARBURG VIRUS IN HUMAN ENDOTHELIAL CELLS A POSSIBLE MECHANISM FOR THE DEVELOPMENT OF VIRAL HEMORRHAGIC DISEASE" JOURNAL OF CLINICAL INVESTIGATION, NEW YORK, NY, US, vol. 91, no. 4, April 1993 (1993-04), pages 1301-1309, XP001028245 ISSN: 0021-9738
- GUBLER D J: "THE GLOBAL PANDEMIC OF DENGUE/DENGUE HAEMORRHAGIC FEVER: CURRENT STATUS AND PROSPECTS FOR THE FUTURE" ANNALS OF THE ACADEMY OF MEDICINE, SINGAPORE, ACADEMY OF MEDICINE, SG, vol. 27, no. 2, March 1998 (1998-03), pages 227-234, XP000952639 ISSN: 0304-4602

## Description

The present invention relates to the use of inhibitors (small molecular weight compounds) of the protein kinases MEK according to claim 1 especially inhibitors of the protein kinase MEK1, for manufacture of a medicament for prophylaxis or treatment of virally induced hemorrhagic fever or hemorrhagic shock syndromes, and for inhibiting virally induced TNF-α and IL-1β production.

### Introduction

Cytokines are soluble glycoproteins (proteins coated with sugars) released by cells of the immune system. The immune system is a collection of cells (such as B-Cells, T-Cells, etc.), chemical messengers (e.g. cytokine) and proteins (such as immunoglobulin) that work together to protect the body from potentially harmful, infectious microorganisms, such as bacteria, viruses and fungi.

Human leukocyte antigens (HLA) are proteins located on the surface of white blood cells which play an important role in our body's immune response to foreign substances. TNF-α is produced predominantly by macrophages and some other cells. Macrophages are large white blood cells, derived from monocytes (a subclass of mononuclear leukocytes). Monocytes are white blood cells which can ingest dead or damaged cells (through phagocytosis) and provide immunological defenses against many infectious organisms. Monocytes migrate into tissues and develop into macrophages.

Cytokines like IL1 β and TNF-α act alone or together to induce systemic inflammation (e.g., fever). LPS (Lipopolysaccharide), an endotoxin from bacterial cell wall, stimulates production of TNF-α. TNF-α is also chemotactic for neutrophils and monocytes. Neutrophils are leukocytes (white blood cells) of the polymorphonuclear leukocyte subgroup. Neutrophils form a primary defense against bacterial infections.

Shock means a life-threatening condition where blood pressure is too low to sustain life. A shock occurs when a low blood volume (due to severe bleeding, excessive fluid loss or inadequate fluid uptake), inadequate pumping action of the heart or excessive dilation of the blood vessel walls (vasodilation: excessive relaxation or dilation of the blood vessel walls) causes low blood pressure. This in turn results in inadequate blood supply to body cells, which can quickly die or be irreversibly damaged.

### Tumor Necrosis Factor

The first suggestion that a tumor necrotizing molecule existed was made when it was observed that cancer patients occasionally showed spontaneous regression of their tumors following bacterial infections (Coley, W.B. (1981) Ann. Surg. 14:199). Subsequent studies in the 1960s indicated that host-associated (or endogenous) mediators, manufactured in response to bacterial products, were likely responsible for the observed effects (Beutler, B. & A. Cerami (1989) Annu. Rev. Immunol. 7:625; O'Malley, W.E. *et al.* (1962) J. Natl. Cancer Inst. 29:1169). In 1975, it was shown that a bacterially-induced circulating factor had strong anti-tumor activity against tumors implanted in the skin in mice (Carswell, E.A. *et al.* (1975) Proc. Natl. Acad. Sci. USA 72:3666). This factor, designated tumor necrosis factor (TNF), was subsequently isolated (Aggarwal, B.B. *et al.* (1985) J. Biol. Chem. 260:2345), cloned (Pennica, D. *et al.* (1984) Nature 312:724), and found to be the prototype of a family of molecules that are involved with immune regulation and inflammation (Gruss, H-J. & S.K. Dower (1995) Blood 85:3378; Cosman, D. (1996) In *Blood Cell Biochemistry, Vol. 7:Hematopoietic Cell Growth Factors and Their Receptors,* Whetten, A.D. and J. Gordon, eds., Plenum Press, New York). The receptors for TNF and the other members of the TNF superfamily also constitute a superfamily of related proteins (Baker, S.J. & E.P. Reddy (1996) Oncogene 12:1; Lotz, M. *et al.* (1996) J. Leukoc. Biol. 60:1; Armitage, R.J. (1994) Curr. Opin. Immunol. 6:407; Ware, C.F. *et al.* (1996) J. Cell. Biochem. 60:47).

**TNF**-α: Human TNF-α is a 233 aa residue, non-glycosylated polypeptide that exists as either a transmembrane or soluble protein (Shirai, T. *et al.* (1985) Nature 313:803; Wang, A.M. *et al.* (1985) Science 228:149). When expressed as a 26 kDa membrane bound protein, TNF-α consists of a 29 aa residue cytoplasmic domain, a 28 aa residue transmembrane segment, and a 176 aa residue extracellular region. The soluble protein is created by a proteolytic cleavage event via an 85 kDa TNF-α converting enzyme (TACE), which generates a 17 kDa, 157 aa residue molecule that normally circulates as a homotrimer (Kreigler, M. *et al.* (1988) Cell 53:45; Smith, R.A. & C. Baglioni (1987) J. Biol. Chem. 262:6951). Normal levels of circulating TNF are reported to be in the 10-80 pg/mL range (Steinshamn, S. *et al.* (1995) Br. J. Haematol. 89:719; Spengler, U. *et al.* (1996) Cytokine 8:864). While both membrane-bound and soluble TNF-α are biologically active, soluble TNF-α is reported to be more potent (Decoster, E. *et al.* (1995) J. Biol. Chem. 270:18473). Mouse to human, full-length TNF-α, shows 79% aa sequence identity (Pennica, D. *et al.* (1985) Proc. Natl. Acad. Sci. USA 82:6060 ; Fransen, L. *et al.* (1985) Nucleic Acids Res. 13:4417). The variety of cell types known to express TNF-α is enormous and includes macrophages, CD4⁺ and CD8⁺ T cells (Ware, C.F. *et al.* (1992) J. Immunol. 149:3881), adipocytes (Kern, P.A. *et al.* (1995) J. Clin. Invest. 95:2111), keratinocytes (Lisby, S. *et al.* (1995) Int. Immunol. 7:343), mammary and colon epithelium (Varela, L.M. & M.M. (1996) Endocrinology 137:4915; Jung, H.C. *et al.* (1995) J. Clin. Invest. 95:55), osteoblasts (Modrowski, D. *et al.* (1995) Cytokine 7:720), mast cells (Bissonnette, E.Y. *et al.* (1995) Immunology 86:12), dendritic cells (Zhou, L-J. & T.F. Tedder (1995) Blood 86:3295), pancreatic beta-cells (Yamada, K. *et al.* (1993) Diabetes 42:1026), astrocytes (Lee, S.C. *et al.* (1993) J. Immunol. 150:2659), neurons (Tchelingerian, J-L. *et al.* (1996) J. Neurosci. Res. 43:99), monocytes (Frankenberger, M. *et al.* (1996) Blood 87:373), and steroid-producing cells of the adrenal zona reticularis (Gonzalez-Hernandez, J.A. *et al.* (1996) J. Clin. Endocrinol. Metab. 81:807).

Tumor necrosis factors alpha and beta are cytokines that bind to common receptors on the surface of target cells and exhibit several common biological activities. TNF-α also shares an important inflammatory property with IL-6 and IL-11, *i.e.* the induction of acute phase reactant protein production by the liver. TNF-α and IL-1β further exert secondary inflammatory effects by stimulating IL-6 synthesis in several cell types. IL-6 then mediates its own effects and those of TNF-α and IL-1β in inducing fever and the acute phase response, thereby perpetuating the inflammatory response through a cascade of cytokines with overlapping properties.

Although in general the effects of cytokines are exerted locally at the site of their production (autocrine and paracrine), TNF-α and TNF- β, as well as IL-1β and IL-6, have major systemic (endocrine) effects when either produced acutely in large amounts, as in the case of bacterial sepsis, or chronically in lesser amounts, as in the case of chronic infections. During sepsis with Gram negative organisms, lipopolysaccharides (endotoxin) released from bacteria trigger the widespread production of TNF-α (and subsequently IL-1β and IL-6) by macrophages. The systemic release of these cytokines has been shown to be responsible for the fever and hypotension that characterize septic shock.

### Viral Hemorrhagic Fever

The term viral hemorrhagic fever (VHF) refers to the illness associated with a number of geographically restricted viruses. This illness is characterized by fever and, in the most severe cases, shock and hemorrhage (Fisher-Hoch SP, Simpson DIH. Dangerous pathogens. Br Med Bull 1985;41: 391-5). A number of other febrile viral infections may produce hemorrhage and the agents of Lassa, Marburg, Ebola, and Crimean-Congo hemorrhagic fevers are known to have caused significant outbreaks of disease with person-to-person transmission.

The increasing volume of international travel, including visits to rural areas of the tropical world, provides opportunity for the importation of these infections into countries with no endemic VHF, such as the United States.

### LASSA FEVER

Lassa virus, named after a small town in northeastern Nigeria, is an enveloped, single-stranded, bisegmented ribonucleic acid (RNA) virus classified in the family Arenaviridae. Its natural host is the multimammate rat Mastomys natalensis. This ubiquitous African rodent lives in close association with humans and is commonly found in and around houses in rural areas. The rats are infected throughout life and shed high levels of virus in their urine. Closely related viruses are found in other areas, but their potential for causing human disease is poorly understood.

Lassa fever was first recognized in 1969 in northern Nigeria. Naturally occurring infections, often associated with subsequent nosocomial outbreaks, have been recognized in Nigeria, Sierra Leone, and Liberia (Monath TP. Lassa fever: review of epidemiology and epizootiology. Bull WHO 1975;52:577-92). Under natural circumstances, infection with Lassa virus occurs through contact with M. natalensis or its excreta. Currently, no vaccine is available for use in humans.

### EBOLA HEMORRHAGIC FEVER

Ebola virus is a single-stranded, unsegmented, enveloped RNA virus with a characteristic filamentous structure. When magnified several thousand times by an electron microscope, these viruses have the appearance of long filaments or threads. Classification of the virus in the new family Filoviridae has been accepted. Ebola has three subtypes (Zaire, Sudan, and Reston) which have common as well as unique epitopes (Kiley MP, Bowen ETW, Eddy GA, et al. Filoviradac: a taxonomic home for Marburg and Ebola viruses. Intervirology 1982;18:24-32). Ebola virus was discovered in 1976 and was named after a small river in northwest Zaire, Africa, where it was first detected. It is morphologically similar to, but antigenically distinct from Marburg virus. The reservoir of the virus in nature remains unknown.

Two epidemics occurred within a short time of each other, the first in southern Sudan (World Health Organization. Ebola hemorrhagic fever in Sudan, 1976: Report of a WHO/International Study Team. Bull WHO 1978;56:247-70) and the second in northwest Zaire (World Health Organization. Ebola hemorrhagic fever in Zaire, 1976: Report of an International Commission. Bull WHO 1978;56:271-93). The mortality rate is between about 30 to 90%.

The mode of acquiring natural infection with Ebola virus is unknown, but secondary person-to-person transmissions are described. The incubation period ranges from 2 to 21 days; the average is approximately 1 week. The illness-to-infection ratio for Ebola virus is unknown. The onset of illness is abrupt, and initial symptoms resemble those of an influenza-like syndrome.

### MARBURG HEMORRHAGIC FEVER

Marburg virus is a single-stranded, unsegmented, enveloped RNA virus that is morphologically identical to, but antigenically distinct from Ebola virus. Classification of the virus in the new family Filoviridae has been accepted. Marburg virus is named after the town in Germany where some of the first cases were described (Martini GA, Siegert R, eds. Marburg virus disease. Berlin: Springer-Verlag). Its reservoir in nature remains unknown.

The mode of acquiring natural infection with Marburg virus is unknown, but secondary spread results from close contact with infected persons or contact with blood or body secretions or excretions are described. The illness-to-infection ratio is unknown but seems high for primary infections.

### CRIMEAN-CONGO HEMORRHAGIC FEVER

Crimean-Congo hemorrhagic fever (CCHF) virus is an enveloped, single-stranded RNA Bunyaviridae. A hemorrhagic fever that had long been recognized in Asia came to international attention after a disease outbreak in the Crimean peninsula in 1944 and 1945 (Hoogstraal H. The epidemiology of tick-borne . Crimean-Congo hemorrhagic fever in Asia, Europe, and Africa. J Med Entomol 1979;15:307-417). The causative agent was later recognized to be identical to the Congo virus (Chumakov MP, Smimova SE, Tkachenko EA. Relationship between strains of Crimean hemorrhagic fever and Congo viruses. Acta Virol 1970;14:82-5), isolated in Zaire, hence the name CCHF. Many wild and domestic animals act as reservoirs for the virus, including cattle, sheep, goats, and hares.

Nosocomial transmission is well described in recent reports from Pakistan, Iraq, Dubai, and South Africa (Swanepoel R, Shepherd AJ, Leman PA, et al. Epidemiologic and clinical features of Crimean-Congo hemorrhagic fever in southern Africa. Am J Trop Med Hyg 1978;36:120-32). The incubation period for CCHF is about 2-9 days.

### DENGU and DENGUE HEMORRHAGIC FEVER

Dengue and dengue hemorrhagic fever (DHF) result from infection by any of four serotypes of dengue viruses. Transmission occurs through the bite of infected Aedes mosquitoes, principally Aedes aegypti, which is also the principal urban vector of yellow fever. Hundreds of thousands of cases of dengue and DHF are reported each year in tropical regions of the Americas, Africa, Asia and Oceania. From 1980 through 1987, 879 632 cases of dengue were reported from countries in the American region ( Pinheiro FP. Dengue in the Americas 1980-1987. Epidemiol Bull 1989;10:1-8). Outbreaks of the more severe form of dengue, DHF, occurred in Cuba in 1981 and in Venezuela in 1989. The majority of DHF cases, however, occur in Southeast Asia. From 1981 through 1986, 796 386 cases of DHF and 9774 deaths caused by dengue were reported from countries in Southeast Asia.

Although dengue is primarily a health problem of tropical areas, one of the earliest dengue epidemics described in the medical literature occurred in Philadelphia in 1780 ( Siler JF, Hall MW, Hitchens AP. Dengue: its history, epidemiology, clinical manifestations, immunity, and prevention. Philippine J Sci 1926;29:1-312). Massive regional efforts to control A. aegypti mosquitoes in the American region during the 1950s and 1960s resulted in the successful (although unfortunately impermanent) eradication of this species from many neighboring countries, but it was never eliminated from the southeastern United States, where it continues to thrive. Outbreaks of indigenous dengue transmission occurred in Texas in 1980 and again in 1986 (Centers for Disease Control. Imported and indigenous dengue fever: United States, 1986. MMWR 1987;33:551-4). Adding to the concern of indigenous dengue transmission is the recent establishment in the United States of another known dengue vector, Aedes albopictus, which was probably imported in shipments of tires from Asia. This species was discovered in Texas in 1985, and focal infestations as far north as Illinois have subsequently been identified. Initially DHF case fatality rates, when the disease was not treated, were as high as 50%.

Object of the present invention is to provide compounds which can be used for manufacture of a medicament for prophylaxis and treatment of hemorrhagic fevers and hemorrhagic shocks, for regulating virally induced TNF-α production, or for treatment of virally induced TNF-α mediated diseases.

This object is solved by the disclosure of the independent claims.

### Description of the invention

According to one aspect, the present invention provides the use of at least one MEK inhibitor, especially a MEK 1 inhibitor selected from 2-(2-chloro-4-iodophenylamino)-N-cyclopropylmethoxy-3,4-difluorobenzamide, or 1,4-diamino-2,3-dicyano-1,4-bis(2-aminophenylthio)butadiene, for the manufacture of a medicament for prophylaxis or treatment of virally induced hemorrhagic fever and/or hemorrhagic shock syndromes.

Surprisingly it was found that hemorrhagic fevers and hemorrhagic shocks only induced by viruses can be treated with the presently claimed MEK inhibitors while bacterially induced hemorrhagic fevers or hemorrhagic shocks show no significant effect during treatment with the presently claimed MEK inhibitors.

**Shock** refers to a state in which adequate perfusion to sustain the physiologic needs of organ tissues is not present. Shock and shock-like states may be produced by many conditions, including sepsis, blood loss, impaired autoregulation, and loss of autonomic tone.

In **hemorrhagic shock**, blood loss occurs that exceeds the body's ability to compensate and provide adequate tissue perfusion and oxygenation. This frequently is due to trauma, but it may be caused by spontaneous hemorrhage, surgery, and a host of other causes.

Failure of compensatory mechanisms in hemorrhagic shock can lead to death. Without intervention, a classic trimodal distribution of deaths is seen in severe hemorrhagic shock. An initial peak of mortality occurs within minutes of hemorrhage due to immediate exsanguination. Another peak occurs after 1 to several hours due to progressive decompensation. A third peak occurs days to weeks later due to sepsis and organ failure.

Increased permeability of endothelial cells leads to a visceral effusions, pulmonary interstitial edema, and renal tubular dysfunction, which are a component of the shock seen in patients with filovirus infection. Said shock is presumably due to substances such as tumor necrosis factor α (TNF-α) which may increase vascular permeability (Schnittler HJ, Mahner F, Drenckhahn D, Klenk HD, Feldmann H., Replication of Marburg virus in human endothelial cells: a possible mechanism for the development of viral hemorrhagic disease. J Clin Invest 1993;91:1301-1309). Studies using tumor necrosis factor alpha show that it is the same mediators that result in the increased endothelial permeability as well as the production of shock.

**Viral hemorrhagic fevers** comprise, as discussed above in detail, Ebola hemorrhagic fever, Marburg hemorrhagic fever, Lassa fever, Crimean-Congo hemorrhagic fever (CCHF), and dengue hemorrhagic fever (DHF).

Viral hemorrhagic fevers are caused by viruses from four families: filoviruses, arenaviruses, flaviviruses, and bunyaviruses. The severity of viral hemorrhagic fever can range from a relatively mild illness to death. The usual hosts for most of these viruses are rodents or arthropods (such as ticks and mosquitoes). In some cases, the natural host for the virus is not known.

People can get Ebola hemorrhagic fever by direct contact with virus-infected blood, body fluids, organs, or semen. At present, there is no known cure or treatment. Ebola hemorrhagic fever is one of the deadliest of a group of diseases called viral hemorrhagic fevers. Ebola hemorrhagic fever has occurred in outbreaks in Central Africa. Ebola hemorrhagic fever is caused by several Ebola viruses. The source of these viruses in nature is not known. Depending on the virus, the disease can get worse until the patient becomes very ill with breathing problems, severe bleeding (hemorrhage), kidney problems, and shock.

Marburg hemorrhagic fever and Ebola hemorrhagic fever are the most interesting diseases within said group of viral hemorrhagic fevers. Marburg hemorrhagic fever is a severe type of viral hemorrhagic fever which affects both humans and non-human primates. Caused by a genetically unique zoonotic (that means, animal-borne) RNA virus of the filovirus family, its recognition led to the creation of this virus family.

Preferred is the use of the MEK inhibitors, especially MEK1 inhibitors according to claim 1, of the present invention for the prophylaxis or treatment of hemorrhagic fever and/or hemorrhagic shock syndromes induced by a filovirus, a arenavirus, a flavivirus, or a bunyavirus. Most preferred is the use of said MEK inhibitors for the prophylaxis or treatment of hemorrhagic fever and/or hemorrhagic shock syndromes induced by a filovirus.

The filovirus family comprises the Marburg virus, Ebola virus and Reston virus. Marburg virus isolates appear to belong to a single species, but there are three known subtypes of Ebola that differ significantly from each other (Ellis DS, Starnford S, Tovey DG, et al. Ebola and Marburg viruses: II. Their development within vero cells and the extra-cellular formation of branched and torus forms, J. Med. Chem. 1979, 4, 213-225). Comparison of 1172 nucleotides from the GP gene shows more than a 40% difference between any pair of the three subtypes from Sudan, Zaire, and Reston.

The **Ebola virus** is a bacilliform rod that contains a negative-sense RNA genome (Sanchez A, Kiley MP. Identification and analysis of Ebola virus proteins. Virology 1985;147:169). Various animals including monkeys, guinea pigs, suckling mice, and hamsters have been infected with Ebola virus. From the three subtypes of Ebola, the Zaire subtype is highly virulent and usually leads to death, the Sudan subtype often causes a self-limited infection in mice, guinea pigs, and occasionally in monkeys, while the Reston subtype is less pathogenic for monkeys and guinea pigs compared to the other subtypes.

The **Marburg virus** replicates in human monocytes/macrophages, resulting in cytolytic infection and release of infectious virus particles. Replication also leads to intracellular budding and accumulation of viral particles in vacuoles, thus providing a mechanism by which the virus may escape immune surveillance. Monocytes/macrophages are activated by Marburg virus infection as indicated by TNF-α. TNF-α is a major immune response-modifying cytokine produced primarily by activated macrophages. Like IL-1β, TNF-α induces the expression of other autocrine growth factors, increases cellular responsiveness to growth factors and induces signaling pathways that lead to proliferation. TNF-α acts synergistically with EGF and PDGF on some cell types. Like other growth factors, TNF-α induces expression of a number of nuclear proto-oncogenes as well as of several interleukins. Supernatants of monocyte/macrophage cultures infected with Marburg virus increase the permeability of cultured human endothelial cell monolayers. The increase in endothelial permeability correlates with the time course of TNF-α release and can be inhibited by a TNF-α specific monoclonal antibody. Furthermore, recombinant TNF-α added at concentrations present in supernatants of virus-infected macrophage cultures increases endothelial permeability indicating that TNF-α plays a critical role in mediating increased permeability, which was identified as a paraendothelial route shown by formation of interendothelial gaps. Surprisingly, the combination of viral replication in endothelial cells and monocytes/macrophages and the permeability-increasing effect of virus-induced cytokine release provide the first experimental data for a novel concept in the pathogenesis of viral hemorrhagic fever.

The inhibitors of the protein kinases MEK according to claim 1 are administered to a subject in need in a dosage corresponding to an effective concentration in the range of 5 nM to 10 µM, preferably in a range of 50 nM to 1 µM.

In 1990, mammalian p44 MAPK was cloned and referred to as extracellular signal-regulated kinase (ERK1). Since the initial discovery of ERK1, 12 MAPK genes encompassing five subfamilies have been identified in mammalian cells that are defined by sequence homology and functional similarity (cf. Table 1). The MAPK family members include ERK1/2, p38alpha, beta, gamma and delta, JNK1, 2, 3, ERK3, 4 and 5.

**Table 1**

| **Acronym** | **Name** |
|---|---|
| **MKKKKs and MKKKs** | |
| Raf1, A- and B-raf | |
| MEKK1, 2, 3 and 4 | MAPK/ERK kinase kinase 1-4 |
| MAPKKK5/ASK-1 | MAP kinase kinase kinase5/apoptosis-signal regulating kinase-1 |
| MLK1, 2 and 3 | Mixed lineage kinase 1-3 |
| **MKKs** | |
| MEK1 and 2 | MAPK/ERK kinase |
| JNKK | JNK kinase (also known as MKK4 or SEK-1) |
| MKK3, 5, 6, 7 | MAPK kinase |
| **MAPKs** | |
| ERK1,2,3,4 and 5 | Extracellular-signal regulated kinase |
| JNK1,2, and 3 | c-jun N-terminal kinase |

There are seven different MKKs that regulate the MAPKs with considerable specificity. MAPK/ERK (MEK) 1 and 2 regulate ERK1/2, whereas JNK kinase (JNKK; also known as SEK-1 or MKK4) and MAPK/ERK kinase 7 (MKK7) regulate JNK activity. MKK3 and MKK6 specifically phosphorylate and regulate p38 activity. MKK5 phosphorylates and regulates ERK5. There are currently 10 different groups of kinases encompassing over 22 different genes that act upstream and regulate the MKKs. One family, the MAPK/ERK kinase kinases (MEKKs), directly phosphorylate and activate specific MKKs and so are valid MKKKs. Table 1 gives an overview of MAP kinases and their acronyms.

Tumor necrosis factor alpha has multiple biological functions including the prolonged activation of the *collagenase* and *c-Jun* genes, which are regulated via their AP-1 binding sites. Incubating human fibroblasts with TNF-α induces prolonged activation of JNK, the c-Jun kinase, which phosphorylates the transactivation domain of c-Jun. Furthermore, an immune complex kinase assay specifically demonstrates that TNF-α stimulates the activity of JNK1. TNF-α also produces a small and transient increase in extracellular signal-regulated kinase (ERK) activity, but no measured increase in Raf-1 kinase activity.

The activation of JNK by TNF-α does not correlate with Raf-1 or ERK activity. The kinetics of Raf-1, ERK, and JNK induction by epidermal growth factor, phorbol 12-myristate 13-acetate, or TNF-α indicate distinct mechanisms of activation in human fibroblasts. Tumor necrosis factor alpha activates the SAPKs (also known as Jun nuclear kinases or JNKs), resulting in the stimulation of AP-1-dependent gene transcription and induces the translocation of NF-kappa B to the nucleus. This results in the stimulation of NF-kappa B-dependent gene transcription. A potential second messenger for these signaling pathways is ceramide, which is generated when TNF-α activates sphingomyelinases.

If TNF-α remains in the body for a long time, it loses its anti tumor activity. This can occur due to polymerization of the cytokine, shedding of TNF receptors by tumor cells, excessive production of anti-TNF antibodies, found in patients with carcinomas or chronic infection, and disruptions in the alpha-2 makroglobulin proteinase system which may deregulate cytokines. Prolonged overproduction of TNF-α also results in a condition known as cachexia, which is characterized by anorexia, net catabolism, weight loss and anemia and which occurs in illnesses such as cancer and AIDS.

Surprisingly it was found that filoviruses induce TNF-α and IL-1β overproduction which may lead to viral hemorrhagic fever and/or hemorrhagic shock syndromes.

In cells that contain TNF receptors, activation of these receptors lead to turning on of many pathways that lead to toxicity in the target cell, and which culminate in apoptosis (regulated self-destruction of the cell). Multiple organ failure is more likely caused by TNF-α induced toxicity than by any other single cause. Neutral sphingomyelinase has been shown to be activated by the TNF receptor, and this, in turn, activates ceramide-activated protein kinase, which then activates the MEK/MAP kinase pathway in the target cells, probably adding to the overall toxic effects of TNF-α. It is known that the MEK/MAP kinase pathway is important in septic shock, and is involved at several vital points in the progression of septic shock. As disclosed in the present invention it was surprisingly found that the virally induced TNF-α, and IL-1β overproduction optionally associated with VHF or hemorrhagic shock syndroms can be regulated using MEK inhibitors, preferably MEK1 inhibitors.

Thus, the present invention is based on the mechanism of action of MEK inhibitors, most preferably MEK1 inhibitors, on the virally induced production, especially overproduction, of TNF-α, and IL-1β which may cause fatal diseases like VHF. Most preferably, the TNF-α and IL-1β overproduction is induced by a Marburg virus, Ebola virus, or Reston virus.

### Pharmaceutical Compositions

The present invention relates to the use of at least one of the compounds 2-(2-chloro-4-iodophenylamino)-N-cyclopropylmethoxy-3,4-difluorobenzamide, or 1,4-diamino-2,3-dicyano-1,4-bis(2-aminophenylthio) butadiene, for the preparation of a pharmaceutical composition for the prophylaxis and/or treatment of virally induced hemorrhagic fever and/or hemorrhagic shock syndromes.

The MEK inhibitors according to claim 1 can also be administered in form of their pharmaceutically active salts optionally using substantially nontoxic pharmaceutically acceptable carrier, excipients, adjuvents or diluents. The medications of the present invention are prepared in a conventional solid or liquid carrier or diluent and a conventional pharmaceutically-made adjuvant at suitable dosage level in a known way. The preferred preparations are in administratable form which is suitable for oral application. These administratable forms, for example, include pills, tablets, film tablets, coated tablets, capsules, powders and deposits. Other than oral administratable forms are also possible. The MEK inhibitors or pharmaceutical preparations containing said MEK inhibitors may be administered by any appropriate means, including injection (intravenous, intraperitoneal, intramuscular, subcutaneous) by absorption through epithelial or mucocutaneous linings (oral mucosa, rectal and vaginal epithelial linings, nasopharyngial mucosa, intestinal mucosa); orally, rectally, transdermally, topically, intradermally, intragastrally, intracutanly, intravaginally, intravasally, intranasally, intrabuccally, percutanly, sublingually, or any other means available within the pharmaceutical arts.

Liquid form preparations include solutions, suspensions and emulsions. As an example may be mentioned water or water-propylene glycol solutions for parenteral injections or addition of sweeteners and opacifiers for oral solutions, suspensions and emulsions. Liquid form preparations may also include solutions for intranasal administration.

Aerosol preparations suitable for inhalation may include solutions and solids in powder form, which may be in combination with a pharmaceutically acceptable carrier such as inert compressed gas, e.g. nitrogen.

Techniques for the formulation and administration of the MEK inhibitors of the present invention may be found in "Remington's Pharmaceutical Sciences" Mack Publishing Co., Easton PA. A suitable composition comprising at least one MEK inhibitor, especially MEK1 inhibitor, of the invention may be a solution of the compound in a suitable liquid pharmaceutical carrier or any other formulation such as tablets, pills, film tablets, coated tablets, dragees, capsules, powders and deposits, gels, syrups, slurries, suspensions, emulsions, and the like.

A therapeutically effective dosage of a MEK inhibitor according to claim 1 refers to that amount of the compound that results in an at least partial inhibition of viral mediated TNF-α release. Toxicity and therapeutic efficacy of such compounds can be determined by standard pharmaceutical, pharmacological, and toxicological procedures in cell cultures or experimental animals for determining the LD50 (the dose lethal to 50% of the population) and the ED50 (the dose therapeutically effective in 50% of the population). The dose ratio between toxic and therapeutic effect is the therapeutic index and can be expressed as the ratio between LD50 and ED50. The dosage of the compound lies preferably within a range of circulating concentrations that include the ED50 with little or no toxicity. More preferably, the dosage of the compound corresponds to an effective concentration in the range of 0.05 - 10 µM. The actual amount of the composition administered will be dependent on the subject being treated, on the subject's weight, the severity of the affliction, the manner of administration and the judgement of the prescribing physician.

### Description of the Figures:

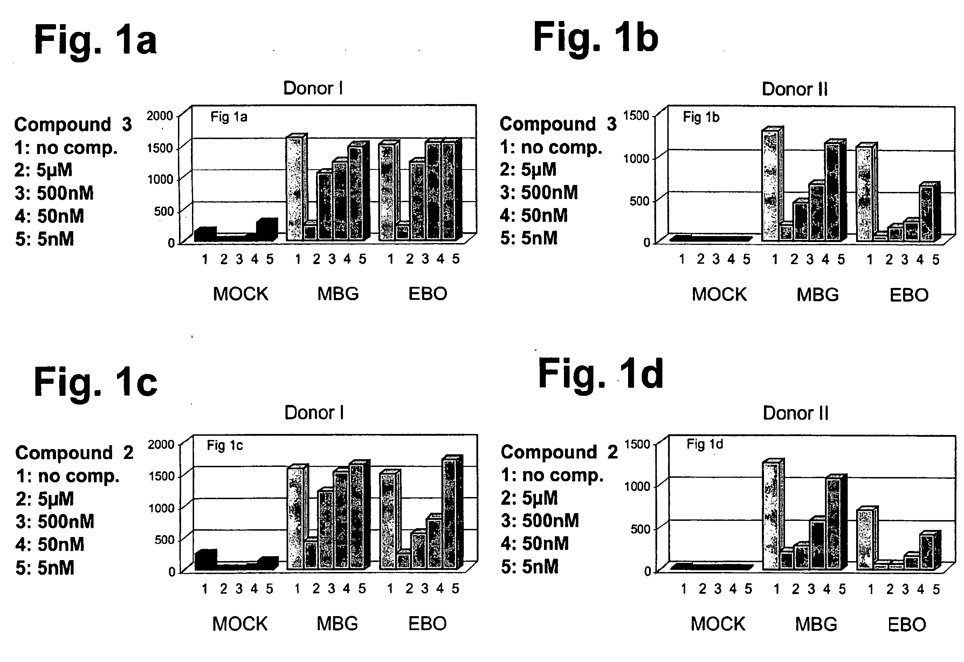

- Figs. 1a to 1d: show the effect of two MEK inhibitors at different concentrations on the TNF-α release of Ebola (EBO) or Marburg (MBG) virus infected peripheral blood mononuclear cells (PBMC) of two different donors. As a control, the above-mentioned PBMC's were incubated with the supernatants of non virus-producing Vero E6 cells (MOCK). 21 hours post infection (pi), mock-infected PBMC's did not release TNF-α in significant amounts to the supernatants irrespective of absence or presence of the inhibitor (Figs. 1a to 1d, MOCK columns 1 to 5). Infection of PBMC's with Marburg or Ebola virus in the absence of any inhibitor (positive control) resulted in a dramatic release of TNF-α to the supernatant 21 h post infection (Figs. 1a to 1d, MBG column 1; Figs. 1a to 1d, EBO column 1). In the presence of the respective inhibitor TNF-α release to the supernatant was dramatically inhibited in a dose-dependant manner between 5 nM to 5 µM concentrations (Figs. 1a to 1d, MBG columns 2 to 5; Figs. 1a to 1d, EBO columns 2 to 5). The effect was observed for both donors. These results suggest, that both compounds can efficiently inhibit TNF-α release induced by Ebola and Marburg viruses.

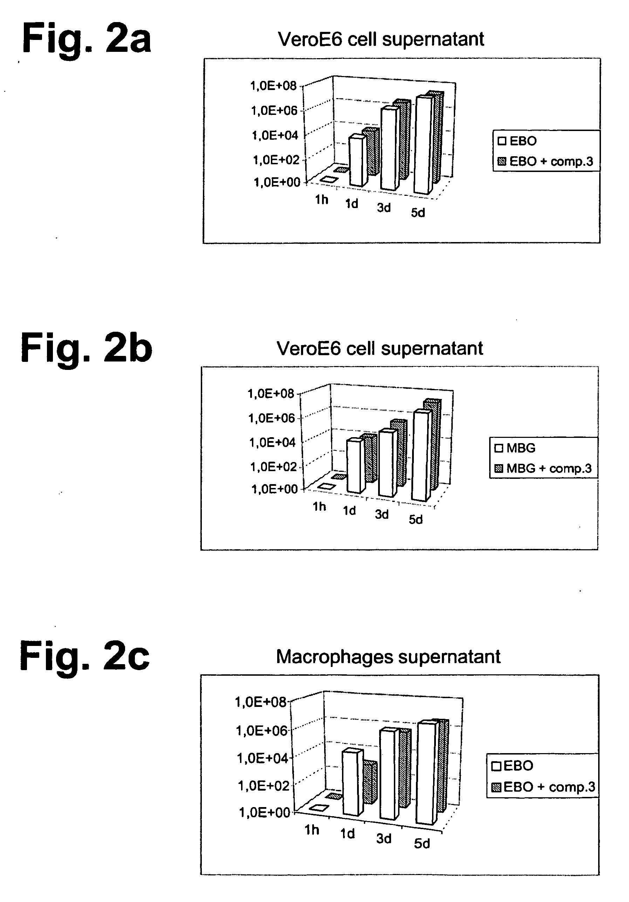

- Figs. 2a to 2c: demonstrate no inhibitory effects of the MEK-inhibitor 1,4-diamino-2,3-dicyano-1,4-bis(2-aminophenylthio)butadiene (compound 3) on replication of Ebola and Marburg virus in a plaque test in VeroE6 cells (Figs. 2a and 2b) and human macrophages (Fig. 2c). Presence or absence of compound 3 does not have an obvious effect on filovirus replication in the above mentioned cells reflected by the presence of similar amounts of plaques 5 days post infection.

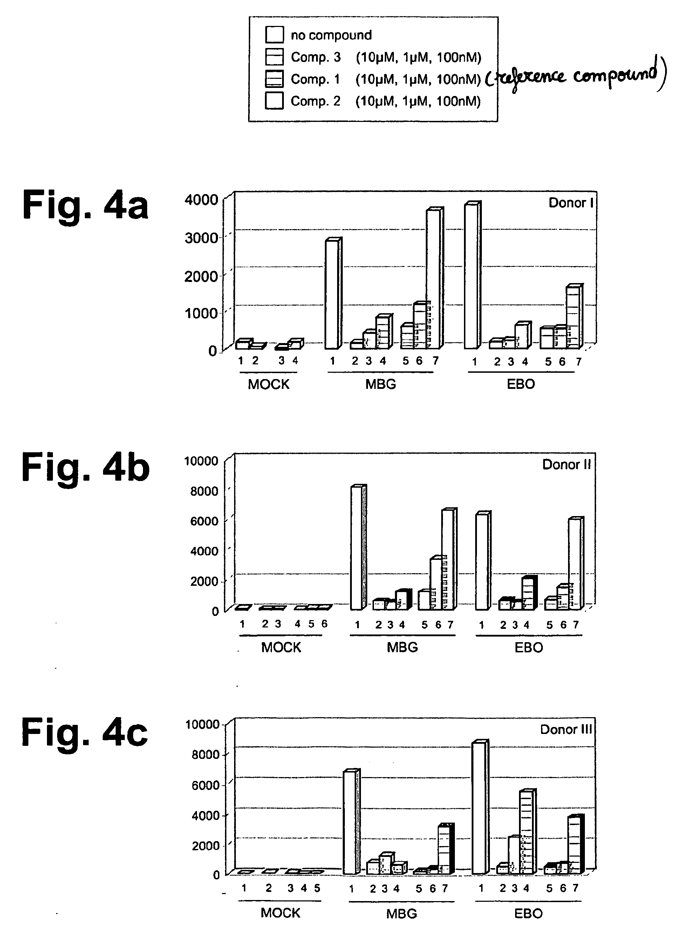

- Fig. 3: shows the inhibitory effect of the MEK-inhibitor 1,4-diamino-2,3-dicyano-1,4-bis(2-aminophenylthio)butadiene (compound 3) on the release of IL-1β, a cytokine commonly upregulated in inflammatory processes. IL-1β is not detected in mock-infected VeroE6 cells despite presence (Fig. 3 Mock + column) or absence (Fig. 3 MOCK - column) of the MEK-inhibitor compound 3. While infection of VeroE6 cells with Marburg or Ebola virus results in a prominent increase of IL-1β 22 h post infection in the supernatant of the cells (Fig. 3 MBG "-" column; fig 3 EBO-Z "-" column), presence of the MEK-inhibitor compound 3 dramatically reduces the release of IL-1β to the supernatant (Fig. 3 MBG "+" column; fig 3 EBO "+" column).
- Figs. 4a to 4c: show the dose-dependent inhibitory effect of compounds 1 (reference compound) and 3 (Fig. 4a) and the inhibitory effect of an additional MEK-inhibitor (2-(2-chloro-4-iodophenylamino)-N-cyclopropylmethoxy -3,4-difluoro-benzamide, compound 2; Figs. 4b and 4c) at different concentrations on the TNF-α release of Ebola (EBO) or Marburg (MBG) virus infected peripheral blood mononuclear cells (PBMC) of three different donors 20 hours post infection.

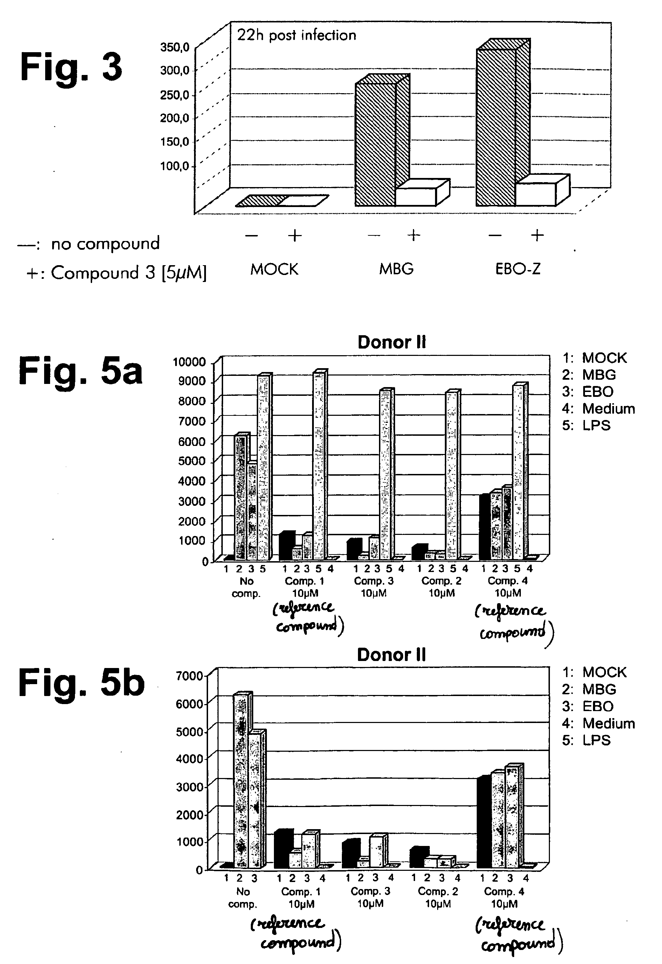

- Figs. 5a to 5b: demonstrate the inhibitory effects of the reference compound 2'-amino-3'-methoxyflavone (compound 1), 2-(2-chloro-4-iodophenylamino)-N-cyclopropylmethoxy-3,4-difluorobenzamide (compound 2), and 1,4-diamino-2,3-dicyano-1,4-bis(2-aminophenylthio)butadiene (compound 3) on the virally (MBG Marburg virus; EBO Ebola virus) induced TNF-α release in primary human macrophages from two donors, 17 hours after viral induction. In sharp contrast, the compounds 1, 2 and 3 did not reduce bacterial LPS-induced TNF-α release (see column 5 in Fig. 5a) in comparison to viral induced TNF-α release (see columns 2 and 3 in Fig. 5a). The reference compound 4, the p38 kinase inhibitor 4-(4-fluorophenyl)-2-(4-hydroxyphenyl)-5-(4-pyridyl)-1H-imidazole did not significantly inhibit Marburg or Ebola virus induced TNF-α release at usually effective concentrations (Fig. 5a last five columns numbers 2 and 3). Fig. 5b represents magnifications of the viral data from Fig. 5a without the data for the bacterial LPS-induced TNF-α release.

The reaction conditions of the tests shown in the Figures were as follows: The cells were incubated with the inhibitor 30 minutes before the respective viral infection or stimulation with lipopolysaccaride (LPS), respectively. The temperature was 37°C in each case.

In the Figures, the units given in the graphs refer to picogram TNF-α per ml and IL-1β, respectively, in the supernatant with cells.

The "medium" designated in the Figures is a RPMI 1640 medium supplemented with 2 mM glutamine, 100 U/ml penicillin, 100 µg/ml streptomycin, 2 mM non-essential amino acids, 2 mM pyruvate, and 5% human AB serum.

With the term "Mock", supernatants of VeroE cells not infected with Marburg-Virus and Ebola-Virus cells are denoted. The Mock tests were run under the same conditions as the tests with the MBGV and EBOV cells, i.e. in the same medium (DMEM, 2% FCS) and for the same incubation period.

"LPS" means lipopolysaccaride from E. coli and obtained from Sigma, Deisenhofen, Germany. LPS was used as a positive control for TNF-α expression based on bacterial stimulation.

### Examples

### Materials and Methods

Primary human peripheral blood mononuclear cells were prepared from buffy coats of healthy donors. The mononuclear cell fraction was separated and finally resuspended in RPMI 1640 medium supplemented with 5% heat-inactivated pooled human serum. For separation of adherent monocytes/macrophages from non-adherent cells, 5 x 10⁶ cells were plated into 24-well tissue culture plates, incubated for 1 h at 37°C, and subsequently washed to remove non-adherent cells. Adherent cells were cultured for 7 days in RPMI 1640 medium containing 5% human serum in order to obtain a macrophage-like morphology. Monocytes/macrophages were infected with virus strains at an MOI of 10 after 7 days in cell culture. Adsorption of viral particles was performed for 1 h at 37°C. Subsequently, the inoculum was removed, and the cells were washed twice with phosphate-buffered saline (PBS). RPMI 1640 medium supplemented with 5% human serum from healthy donors and the respective concentrations of various chemical inhibitors was added, and incubation proceeded for 12, 17, or 24h, respectively, at 37°C. After incubation times, tissue culture supernatants were collected and determination of TNF-α in the culture supernatants was performed with an enzyme-linked immunosorbent assay (ELISA) according to the manufacturer's protocol.

*Viruses and cell lines:* In this study we used the Musoke strain of MBGV, the Mayinga strain of the Zaire species of EBOV (EBOV-Zaire), and the Z-strain of Sendai virus. MBGV and EBOV virus stocks were kindly provided by the Special Pathogens Branch, Centers for Disease Control and Prevention, Atlanta, USA. MBGV and EBOV stocks were freshly prepared in Vero E6 cells (ATCC 1568). Mock-infected Vero E6 cells were treated the same way in order to prepare a control (mock stock). Vero E6 cells were cultured in Dulbecco's Minimum Essential Medium (DMEM) (GIBCO-BRL, Germany) supplemented with 10% fetal calf serum (Biochrom, Germany), penicillin (100 U/ml), streptomycin (100 µg/ml) and L-glutamine (2 mM). For virus propagation DMEM with 2% fetal calf serum was used.

Sendai virus were amplified in the allantoic cavity of 11-day-old embryonated chicken eggs.

*Endotoxin test* Prior to use, all virus stocks and media were analyzed for endotoxin presence using the 'Limulus amebocyte lysate test' (QCL-1000; BioWhittaker, Walkersville, MD. U.S.A.). All compounds and media used in this study contained less than 0.3 EU/ml which was less or equivalent to the amounts found in the mock stock used as controls for the experiments.

*Isolation of peripheral blood mononuclear cells (PBMC):* Human PBMCs were obtained from leukocyte-rich buffy coats of healthy donors (Marburg, Germany). Cells in fresh, single buffy coats were fractionated by centrifugation on Ficoll-Paque gradient (Pharmacia), and blood mononuclear cells (2 x 10⁷ cells/well) were allowed to adhere onto 24-well plates (Primaria, Falcon) for 1h in RPMI 1640 medium supplemented with glutamine (2 mM), penicillin (100 U/ml), streptomycin (100 µg/ml), nonessential amino acids (2 mM), pyruvate (2 mM), and 5% human AB-serum. Plastic-adhered monocytes were washed with PBS and differentiated into macrophages by culturing them for 7 days at 37°C in a humidified (95%) 5% CO₂ air atmosphere.

*Inhibitors:* The inhibitors 2'-amino-3'-methoxyflavone (Compound 1), 2-(2-chloro-4-iodophenylamino)-N-cyclopropylmethoxy-3,4-difluorobenzamide (Compound 2), 1,4-diamino-2,3-dicyano-1,4-bis(2-aminophenylthio)butadiene (Compound 3), and 4-(4-fluorophenyl)-2-(4-hydroxyphenyl)-5-(4-pyridyl)-1H-imidazole (Compound 4) have been obtained from Calbiochem and were dissolved in dimethyl sulphoxide. Compounds 1 and 4 are reference compounds.

*Assaying of cytotoxicity:* The cytotoxic effect of the inhibitors was measured by the MTT-assay as described elsewhere. Twenty four , forty eight, and seventy two hours after the final treatment, 100 µl of MTT reagent (Sigma) was added to each sample for 4 h, then 100 µl of solubilization solution (20% SDS/50% DMF) was added to the cells for 14h. Plates were analyzed on a microplate reader at 595 nm.

*Infection and treatment of macrophages :* The infection was performed on day 7 post seeding at a MOI of 10. Where indicated cells were incubated with the appropriate inhibitor 30 min prior to infection. After an adsorption period of 1 hour, the inoculum was replaced by new medium (RPMI containing 5% human AB-serum) and the cultures were incubated for various times at 37°C in a CO₂-incubator (humidity 95%). Where indicated, cells were treated with 2'-amino-3'-methoxyflavone, 2-(2-chloro-4-iodophenylamino)-N-cyclopropylmethoxy-3,4-difluorobenzamide, 1,4-diamino-2,3-dicyano-1,4-bis(2-aminophenylthio)butadiene, 4-(4-fluorophenyl)-2-(4-hydroxyphenyl)-5-(4-pyridyl)-1H-imidazole, respectively, 45min prior to infection or LPS-stimulation. The inhibitors were also present during infection and subsequent incubation periods.

*Detection of Cytokines :* The supernatants from the infected or mock-infected cell cultures were removed, clarified from cell debris by centrifugation (8000 x g, 4°C, 10 minutes) and stored at -80°C. All samples were tested in duplicates for the presence of cytokines after thawing them only once using commercial ELISA systems (human IL-6, TNF-α ELISA Kit: Promocell, human gro-α, IL-1β ELISA: R&D Systems). Since the samples were not inactivated, the analyses were conducted in a biosafety level 4 (BSL4) containment laboratory.

### Western blot analysis

To detect ERK phosphorylation by Western blotting infected or stimulated macrophages were washed with cold PBS and harvested at the indicated times by lysis in cold lysis buffer (10 mM Tris, pH 7.2, 150 mM NaCl, 1% TritonX-100, 1% sodium deoxycholate, 1 mM PMSF, 50 mM sodium fluoride, 1 mM sodium orthovanadate, 50 µg/ml aprotinin, and 50 µg/ml leupeptin). After centrifugation supernatants were assayed for protein by the Amido-Schwarz method as described by Schnittler et al., Replication of Marburg virus in human endothelial cells: a possible mechanism for the development of viral hemorrhagic disease. J Clin Invest 1993;91:1301-1309. Protein samples (1 µg) were analyzed by SDS-polyacrylamide gel electrophoresis on 10% Tris gels. Protein was electrotransferred to Polyvinylidene difluoride membrane (ImmobilonP membrane, Millipore,). Membranes were rinsed in 10 mM Tris-Cl, pH 7.5, 100 mM NaCl, 0,1% Tween-20 (TBS-T), incubated in blocking buffer (TBS-T with 3% BSA) for 1 h and probed with a polyclonal phosphospecific antibody against ERK1 and ERK2 (New England Biolabs) over night at 4°C. Detection of bands was carried out according to the manufacturer's protocol.

Where indicated in the legends to the figures, blots were stripped by incubation at 37°C in *Restore Western Blot Stripping Buffer* (Pierce) for 30 min, followed by washing in TBS-T and blocking before reprobing with an antibody against ERK1 and ERK2 (New England Biolabs).

*Plaque Assay:* Confluent Vero E6 cell monolayers cultured in six-well plates were infected with clarified tissue culture supernatant of MBGV- or EBOV- infected Vero E6 cells and macrophages, respectively, at 10-fold dilutions ranging from 10⁻³ to 10⁻⁸. After 1 hour cells were washed and covered with an overlay of DMEM/1.8% LMP Agarose/2% FCS. Plaques were stained up to 5 days post infection with 0.1% crystal violet in a 10% formaldehyd solution.

### Results

The MEK1 inhibitors 2'-amino-3'-methoxyflavone (reference compound), 2-(2-chloro-4-iodophenylamino)-N-cyclopropylmethoxy-3,4-difluorobenzamide, and 1,4-diamino-2,3-dicyano-1,4-bis(2-aminophenylthio)butadiene respectively, inhibited very strongly and dose-dependently the Marburg virus or Ebola virus induced TNF-α production at least between 100 nM and 1 µM concentrations.

The MEK1 inhibitors 2 -amino-3-methoxyflavone (reference compound), 2-(2-chloro-4-iodophenylamino)-N-cyclopropylmethoxy-3,4-difluorobenzamide, and 1,4-diamino-2,3-dicyano-1,4-bis(2-aminophenylthio)butadiene, or the p38 kinase inhibitor 4-(4-fluorophenyl)-2-(4-hydroxyphenyl)-5-(4-pyridyl)-1H-imidazole (reference compound) had no inhibitory effect at all on the bacterial LPS-induced TNF-α production at 10 µM concentrations.

The 38 kinase inhibitor 4-(4-fluorophenyl)-2-(4-hydroxyphenyl)-5-(4-pyridyl)-1H-imidazole (reference compound) did not significantly inhibit the Marburg virus or Ebola virus induced TNF-α production at usually effective concentrations, indicating that the tested MEK1 inhibitors are powerful and selective inhibitors of filovirus-induced TNF-α production.

## Claims

1. Use of at least one compound selected from:
2-(2-chloro-4-iodophenylamino)-N-cyclopropylmethoxy-3,4-difluorobenzamide,
1,4-diamino-2,3-dicyano-1,4-bis(2-aminophenylthio)-butadiene,
for the manufacture of a medicament for the prophylaxis or treatment of virally induced hemorrhagic fever or hemorrhagic shock syndromes.

2. Use according to claim 1, wherein the hemorrhagic fever or the hemorrhagic shock syndrome are caused by Ebola viruses.

3. Use according to claim 1, wherein the hemorrhagic fever or the hemorrhagic shock syndrome are caused by Marburg virus.

4. Use according to claim 1, wherein the hemorrhagic fever or the hemorrhagic shock syndrome are caused by Lassa virus.

5. Use according to claim 1, wherein the hemorrhagic fever or the hemorrhagic shock syndrome are caused by Crimean-Congo virus.

6. Use according to claim 1, wherein the hemorrhagic fever or the hemorrhagic shock syndrome are caused by Dengue viruses.

## Patentansprüche

1. Verwendung mindestens einer Verbindung ausgewählt aus
2-(2-Chlor-4-iodphenylamino)-N-cyclopropylmethoxy-3,4-difluorbenzamid,
1,4-Diamino-2,3-dicyano-1,4-bis(2-aminophenylthio)-butadien,
zur Herstellung eines Medikaments zur Prophylaxe oder Behandlung viral induzierter hämorrhagischer Fieber-oder hämorrhagischer Schock-Syndrome.

2. Verwendung nach Anspruch 1, wobei das hämorrhagische Fieber- oder das hämorrhagische Shock-Syndrom von Ebola-Viren verursacht sind.

3. Verwendung nach Anspruch 1, wobei das hämorrhagische Fieber- oder das hämorrhagische Shock-Syndrom vom Marburg-Virus verursacht sind.

4. Verwendung nach Anspruch 1, wobei das hämorrhagische Fieber- oder das hämorrhagische Shock-Syndrom vom Lassa-Virus verursacht sind.

5. Verwendung nach Anspruch 1, wobei das hämorrhagische Fieber- oder das hämorrhagische Shock-Syndrom vom Krim-Kongo-Virus verursacht sind.

6. Verwendung nach Anspruch 1, wobei das hämorrhagische Fieber- oder das hämorrhagische Shock-Syndrom von Dengue-Viren verursacht sind.

## Revendications

1. Utilisation d'au moins un composé sélectionné parmi la liste comprenant :
2-(2-chloro-4-iodophénylamino)-N-cyclopropylméthoxy-3,4-difluorobenzamide,
1,4-diamino-2,3-dicyano-1,4-bis(2-aminophénylthio)-butadiène,
dans la fabrication d'un médicament destiné à la prophylaxie ou au traitement de la fièvre hémorragique ou des chocs hémorragiques d'origine virale.

2. Dispositif selon la revendication 1, dans lequel la fièvre hémorragique ou le choc hémorragique est causé(e) par le virus Ebola.

3. Dispositif selon la revendication 1, dans lequel la fièvre hémorragique ou le choc hémorragique est causé(e) par le virus Marburg.

4. Dispositif selon la revendication 1, dans lequel la fièvre hémorragique ou le choc hémorragique est causé(e) par le virus de Lassa.

5. Dispositif selon la revendication 1, dans lequel la fièvre hémorragique ou le choc hémorragique est causé(e) par le virus de la fièvre hémorragique de Crimée-Congo.

6. Dispositif selon la revendication 1, dans lequel la fièvre hémorragique ou le choc hémorragique est causé(e) par les virus de la Dengue.
